Europäisches Patentamt

European Patent Office

Office européen des brevets

(11) Publication number: **0 213 595**
A2

# (12) EUROPEAN PATENT APPLICATION

(21) Application number: 86111822.2

(22) Date of filing: 26.08.86

(51) Int. Cl.⁴: **C 07 K 15/06**
C 07 K 3/02, G 01 N 33/574

(30) Priority: 28.08.85 JP 190336/85

(43) Date of publication of application:
11.03.87 Bulletin 87/11

(84) Designated Contracting States:
DE FR GB

(71) Applicant: Institute of Whole Body Metabolism
340-2, Nauchi, Shiroimachi
Inba-gun Chiba-ken(JP)

(72) Inventor: Hayama, Emiko
6-5-1, Ueno Taito-ku
Tokyo-to(JP)

(72) Inventor: Motoji, Naomi
2-4-4-209, Shimizuguchi Shiroimachi
Inba-gun Chiba-ken(JP)

(72) Inventor: Endo, Yuko
782-1-1103, Higurashi
Matsudo-shi Chiba-ken(JP)

(72) Inventor: Mabuchi, Yasuo
2-308, Nishichibagurinhaitsu 955-1, Sakusabe-cho
Chiba-shi Chiba-ken(JP)

(72) Inventor: Shishido, Akira
2-12-7, Shinmachi Setagaya-ku
Tokyo-to(JP)

(72) Inventor: Shigematsu, Akiyo
2-14-1, 3-chome Akitsu
Narashino-shi Chiba-ken(JP)

(74) Representative: von Kreisler, Alek, Dipl.-Chem. et al,
Deichmannhaus am Hauptbahnhof
D-5000 Köln 1(DE)

(54) New transferrins and use thereof.

(57) A transferrin which is a soluble protein isolated from a biological source, characterized in that: the said transferrin
(a) increases in blood level with development of malignant tumor,
(b) has a molecular weight of about 90,000-100,000 and
(c) has an isoelectric point of about 5.5-6.7
is prepared by embedding malignant tumor cells into an animal followed by recovering the transferin frim serum of the aminal. The said transferrin is useful in early diagnosis of malignant tumor.

EP 0 213 595 A2

## NEW TRANSFERRINS AND USE THEREOF

### FIELD OF THE INVENTION

The present invention relates to transferrins which are useful for the early stage diagnosis of melanoma and other malignant tumors in animal including human, their production, antibodies to the said transferrins and their use in the diagnosis of malignant tumors.

### BACKGROUND OF THE INVENTION

Mechanism of oncogenesis is extremely complicated and it is of urgent necessity for the world to develop a means for detection and micro-analysis of a specific compound from tumor-bearing patients widely usable in early stage diagnosis of various cancer including precancerous conditions. In recent years, some substances such as those represented by carcinoembryonic antigen (CEA) and alpha-fetoprotein have been used for early diagnosis of cancer.

The inventors formerly discovered that a spontaneous melanoma (B-16) can be artifically embedded at any time into liver not only of syngenic mice such as black mice C57BL but also of allogenic animals such as wistar rats or ICR mice (Japanese patent application 11903/1985). This discovery is very surprising because it has generally been considered that implantation of tumor cells is possible only when syngeneic animals are used. In a special example, xenogeneic tumor cells were successfully embedded into immunosuppressive animals such as nude mice which had been treated with radio activity in order to further deppress their immunological response. However, this method cannot be generalized. Accordingly, the above-mentioned inventor's method allowing implantation of melanoma into xenogeneic animal is an entirely new and unprecedented event. Any-time-transplantation of desired tumor into any experimental animal (e.g., albino rats, albino mice, rabbits etc.) was not possible until the said method was presented.

On natively examining by electrophoretic fractionation, soluble proteins (molecular weight: 1,000,000-10,00$\bar{0}$) which was obtained from liver, serum and neoplastic melanoma cell samples of animals growing after prepared by implantation of tumor piece in liver. The inventors surprisingly discovered that there is a significantly increased amount of a protein specific to melanoma in serum, host liver and neoplastic tumor cells. This protein has a molecular weight of about 90,000 and pI of about 6.0 and resembles the normal transferrin of healthy murine except that pI value is slightly higher.

The fact that the above protein (hereinafter, referred to as the specific transferrin) is classified as transferrin is confirmed by amino acid composition analysis using high performance liquid chlomatography (HPLC, Hitachi), by molecular weight and by pI value, which were highly correlated with those of normal human transferrin.

The specific transferrin and its stereoisomers significantly increase in related parts of animal by neoplastic multiplication of melanoma in liver, and implantation of melanoma in liver, the transferrin-producing organ, was found most effective. Moreover, increase of the specific transferrin in serum was also observed with growth of neoplastic tumor when tumor cells in suspension were subcutaneously injected at axillary region of animal within the same pedigree (e.g., B-16 melanoma into C57BL mice),though elevation of the transferrin concentratin was relatively lower than the case of embedding the tumor in the liver.

The specific transferrin, primarily appearing in serum and identified as above, and its analogues (hereinafter, referred to as transferrins), are cleaved into two components, having molecular weights of about 60,000 and 30,000, respectively, by pretreatment using SDS, mercaptoethanol, etc. These facts were confirmed by studies on the transferrins (including stereoisomers) obtained by embedding B-16 melanoma into liver of C57BL mice.

The specific increase of transferrins in serum was observed parallelly in the growth process of various malignant tumor. This fact is very important for developing an early-diagnostic method of cancer.

In particular, the diagnosis of cancer can be easily conducted by collecting serum from patient and quantifying the specific transferrin in the serum sample by conventional radioimunoassay (radioisotope dilution method) over a positive control of the tumor specific transferrin, produced by embedding human melanoma into, e.g., liver of C57BL mice, or an analogue of the said transferrin. Production of monoclonal antibodies to the specific transferrin and their mass-production by genetic engineering are also possible.

### RELATED DISCLOSURES

Elizabeth M. Wilson et al reported that a major protein of the rat Dunning prostate tumor is a transferrin (Cancer Research 42, 243-251, 1982). However, its content is much higher in tumor fluid than in serum (page 245, Table 2). Ian S. Trowbrige et al reported that anti-transferrin receptor monoclonal antibody affect growth of human tumor

- 5 -

cells (Nature 294, 171-173, 1981). Ian S. Trowbrige et al reported that monoclonal antibody to transferrin receptor inhibits human tumor cell growth in vitro (Proc. Natl. Acad. Sci. USA 79, 1175-1179, 1982). International publication WO 85/00812 (Feb. 28, 1985) discloses a complex of a transferrin and antitumor agent.

### SUMMARY OF THE INVENTION

In one aspect, the present invention provides transferrins which are soluble proteins isolated from biological sources, characterized in that: the said transferrins

    (a)   increases in blood level with development of malignant tumor,

    (b)   have molecular weight of about 90,000-100,000, and

    (c)   have isoelectric point of about 5.5-6.7

In another aspect, the present invention provides a process for preparation of the said transferrins which comprises (1) embedding malignant tumor cells into a specific organ, especially liver in the animal, (2) feeding the animal, (3) collecting sera containing the said transferrin from the animal, (4) separating a fraction containing a protein having molecular weight of about 90,000-100,000 and an isoelectric point of about 5.5-5.7 from the said sera and (5) purifying the protein from the said fraction.

In further aspect, the present invention provides a diagnostic agent for malignant tumor comprising at least one of the said transferrins labeled with e.g. an isotope, a fluorophore or an enzyme.

In yet further aspect, the present invention provides antibodies produced in animal in response to at least one of the said transferrins.

In yet further aspect, the present invention provides a kit for detecting malignant tumor comprising a packaged combination of

(a)    a transferrin as defined above, and

(b)    antibodies produced in animal in response to
       an antigen consisting of a transferrin as
       defined above.

       This kit may further comprise

(c)    solvents for reaction and washing,

(d)    tools and/or apparatuses for reaction and
       washing, and/or

(e)    means for detecting label.

DETAILED DESCRIPTION OF THE INVENTION

In the above and subsequent descriptions, suitable examples and illustration of the various definitions are explained in detail as follows.

The term "transferrins" includes the specific transferrin mentioned above and described below and especially in the Examples, other similar transferrins obtainable from different animal sources, stereoisomers thereof and equivalents having the same activity or antigenic determinant.

Preferable transferrins are those containing as amino acid residue,

aspartic acid, threonine, serine, glutamic acid, glycine, alanine, valine, methionine, isoleucine, leucine, tyrosine, phenylalanine, lysine, histidine, arginine and proline.

The specific transferrin is that having an amino acid composition according to ninhydrin method as follows:

| Aspartic acid | 67 |
| Threonine | 34 |
| Serine | 35 |
| Glutamic acid | 62 |
| Glycine | 52 |
| Alanine | 51 |
| Valine | 46 |
| Methionine | 4 |
| Isolencine | 20 |
| Leucine | 54 |
| Tyrosine | 21 |
| Phenylalanine | 35 |
| Lysine | 54 |
| Histidine | 19 |
| Arginine | 26 |

- 8 -

Proline               47

The transferrins have a general charasteristic
that they are glycoproteins containing a terminal sialic
acid residue of poly-mannose type, but their stereochemistry
has not been established.  The transferrins according to the
invention, however, are cleary distinguished from the normal
transferrins of the animals concerned, by the difference in
native two-dimensional electrophoretic pI values.  These
transferrins increase in blood level to a rate represented
by transferrin/albumin of 0.28 - 30, preferably of 0.5 - 20,
more preferably of 0.5 - 20, more preferably of 1.0 - 10 and
most preferably 1.3 - 10 within 20 days after embedding of
maligant cells.

The "label" means an atom or a group of atoms
which emits signals detectable by physical, chemical or
biological means and includes isotopes, particularly
radioisotopes, fluorophores and enzymes.

The "antibodies" include those produced in warm
blood animals, preferably mammals such as monkeys, sheeps,
goats, rabbits and murines.

The "animal" includes warm blood animals such as
mammals and birds, especially cattle, horse, ass, swine,
sheep, goat, rabbit, rat, mouse, fowl, duck etc.

The "body fluid" includes blood especially serum.

The present invention is based upon the discovery
of the transferrins and antibodies thereto, both being defined as above. Accordingly, for other elements therein such as for example, techniques for production, purification, identification, labeling and quantification of the product, as well as solvents, reagents, tools and apparatuses used therein, those known and conventionally used in similar processes may be applied. These can be illustrated as follows.

The production of the transferrins may be conducted by transplanting and growing malignant tumor cells (e.g., melanoma) into the interior of the body (e.g. an organ) of an experimental animal (e.g., mammals such as albino rats, albino mice etc.). Liver is preferred as the host organ because higher production of transferrins is obtainable as compared with the case of embedding the tumor into other organs or tissues. However, since liver has a special vasculature including hematocele, incision of liver causes profuse fleeding, or even if this can be avoided, inflammation associated with edema and abnormal concentration of leucocyte. Thus, growth of neoplastic cells is seriously affected. This disadvantage may be obviated by applying before the implantation a tissue-activating composition comprising fibrinogen, fibronectin, the factor XIII, plasminogen, aprotinine, trombine and calcium chloride on the wound surface according to the description of Japanese application No. 11903/1980. Application of such composition causes activation of phagocytosis, detoxication, immuno reaction and blastema formation, which results in reinforcement of healing and regeneration of the damaged tissue. The production of the antibodies may be conducted by injecting one of the transferrins into an experimental animal according to the conventional immunization method.

As the transferrins and the antibodies accumulate in serum, these can be obtained by treating the serum collected from the animal with a combination of separating means conventionally used in the fractionation of proteins such as for example, salting out, gel chromatography, column chromatography etc. to purify the transferrins and antibodies. The transferrins and antibodies are ordinarily stored as liophylizate. The characterization of the transferrins is made by, e.g., molecular weight, isoelectric point, amino acid composition etc. The characterization of the antibodies is made by the specific reaction with the transferrins. The methods of measuring these values are well known in the art.

The label may be isotope (radioactive) fluorescent agent or enzyme, etc. The radioisotope includes iodine, technetium etc. The method for conjugating an isotope with a protein is known to the art. The fluorescent agent includes fluorescein compounds, rhodamin compounds etc. The enzyme includes β-galactosidase, alkaline phosphatase etc.

Quantitative analysis of the transferrins and antibodies may usually be carried out by utilization of antigen-antibody reaction. Various method are usable and known including homogeneous and heterogeneous processes.

Water is generally used as the solvent which can include pH-buffering agents, stabilizers for proteins (e.g., polysaccharides), preservatives etc. The tools and apparatuses may include flask, bottle, test tube, glass filter etc., which may be ground-in stoppered. The solvents, tools and apparatuses may be selected and combined as necessary and packaged together with the transferrins, the antibodies and, if required, the labels.

Thus, the present invention provides a method of detecting malignant tumors (i.e. cancers) which comprises adding labeled transferrin and antibody to a serum sample and measuring the activity of label on the reaction product of the transferrin and antibody.

The following non-limiting Examples further illustrate the present invention.

Example 1

PRODUCTION OF THE SPECIFIC TRANSFERRIN

B-16 melanoma cells (replicate culture available from National Institute of Cancer) were inoculated into an axillary region of C57BL mice, grown and collected. A suspension of the collected cells was prepared by adding the Hank's solution and centrifuged at 800 rpm for 15 minutes.

- 12 -

The supernatant was discarded. The cell fraction at the bottom of centrifuge tube was treated with a drop of an albumin suspension containing fibrinogen as the main ingredient and a suitable amount of thrombin thereby aggregating after few minutes. The obtained cell aggregate was embedded into liver of C57BL nude mice and SD rats, respectively. Before embedding the cell aggregate, a solution having the following composition was applied onto the incised wound surface of the murine liver.

(A) fibrinogen                          100 mg

    plasma fibronectin                  7 mg

    the factor XIII                    40 U

    plasminogen                        50 $\mu$ g

(B) bovine aprotinin solution

    (3,000 KIU/ml)                      1 ml

(C) bovine thrombin lyophilizate   500 IU

(D) aqueous $CaCl_2$ (40 m mol/l)      1 ml

(Preparation: Before use, (A) is dissolved in (B), (C) is dissolved in (D) separately, and both the solutions are mixed.) After two weeks, sera were collected exhaustively from the animals. The sera were fractionated by using mainly the salting out method together with gel filtration and a fraction having an isoelectric point of 5.5-6.7 and a molecular weight of 90,000-100,000 was further purified to give a transferrin specific to the inoculated B-16 melanoma and irrelative to the animal used for the host. The yield was about 30% based on the protein fraction which was obtained in a yield of 7g from 100ml of the serum.

The contents of the transferrins to albumin (transferrins/albumin) in the serum after inoculating B-16 melanoma cells into the axillary region (a. r.) of C57BL mice and transplanting the tumor mass into liver of C57BL mice were as follows.

Table 1

| days[*] | 3 | 5 | 7 | 14 | 20 |
|------|------|-----|-----|-----|------|
| a.r. | 0.28 | 0.5 | 1.0 | 1.2 | 1.34 |
| liver | 0.28 | 0.5 | 1.2 | 1.4 | 1.55 |

[*]days after inoculation or transplantation

The ratio transferrins/albumin in the liver neoplastic melanoma in the above experiment was as follows.

Table 2

| days | 3 | 5 | 7 | 14 | 20 |
|------|-----|-----|-----|-----|-----|
| ratio | 0.1 | 0.2 | 0.5 | 2.1 | 4.7 |

Example 2

### PRODUCTION OF THE LABELED TRANSFERRIN

The transferrin (10mg) obtained in Example 1 was labeled with $^{125}$I-NaI (100 mCi) by Chloramine-T method to give a transferrin having a specific radioactivity of as high as 5 mCi/10mg.

Example 3

AMINO ACID COMPOSITION OF THE SPECIFIC TRANSFERRIN

The transferrin obtained in Example 1 was analyzed for amino acid composition using Hitachi High Performance Amino Acid Analyzer 835(ninhydrine method, 4φX 150, 120min. cycle). The result is shown below.

Reference

C57BL (normal human)

| | | |
|------|----|----|
| Asp | 67 | 71 |
| Thr | 34 | 25 |
| Ser | 35 | 35 |
| Glu | 62 | 53 |
| Gly | 52 | 46 |
| Ala | 51 | 51 |
| Val | 46 | 40 |
| Met | 4 | 8 |
| Ile | 20 | 14 |
| Leu | 54 | 52 |
| Tyr | 21 | 24 |
| Phe | 35 | 27 |
| Lys | 54 | 49 |
| His | 19 | 17 |
| Arg | 26 | 23 |
| Pro | 47 | 36 |

Example 4

ELECTROPHORESIS OF THE SPECIFIC TRANSFERRIN

Two-dimensional micro-electrophoresis of the specific transferrin was carried out in which the first dimension was isoelectric electrophoresis. Each serum sample (1μl) was charged on 4% polyacrylamide gel containing carrier ampholyte Ampholin (pH 3.5-10, LKB Produkter, AB), 8 samples per run, migrated at 8mA constant current up to 300V and then at 300V constant voltage for 30 minutes. The second dimension was carried out with 4-17% gradient polyacrylamide gel and tris glycine buffer (pH 8.3) migrating for 2 hours. Immediately after the migration, the migrated carrier was stained with a staining solution containing 0.1% CBB R-250/50% methanol/7% acetic acid and then destained with 7% acetic acid.

Conditions for SDS electrophoresis was as follows.

The sample was treated with SDS. A portion (about 10-20μl) of the sample was migrated in 12% polyacrylamide gel containing 0.1% SDS and tris glycine buffer (containing 0.1% SDS) at 40mA for 6 hours.

As a result, a tumor transferrin content in blood could be determined easily, exactly and rapidly.

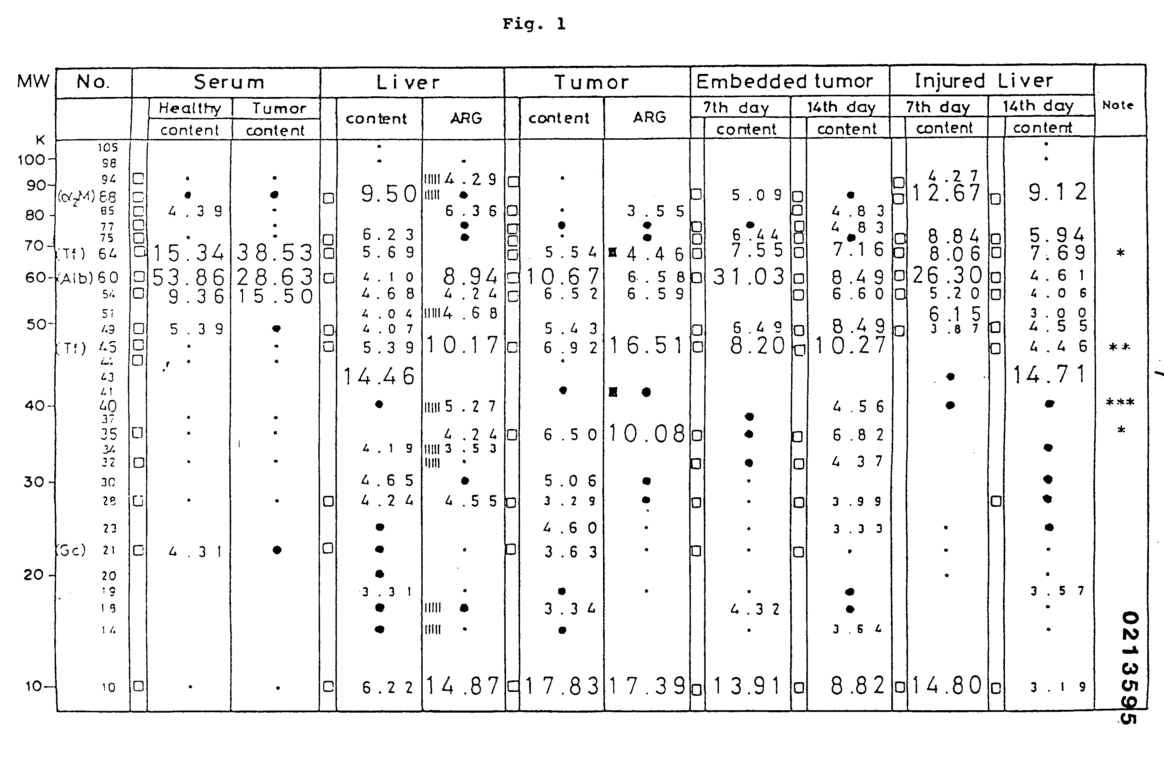

The result is shown in Fig. 1. Abbraviations in Fig. 1 are as follows. □: correspondent to those of the serum (quantitatively), ‖‖‖: showing negative autoradiographic image of the tumor but positive of the liver, ▨: showing negative autoradiographic image of the liver but positive of the tumor, ●: existence (less than 3%) of the target protein,•: faint detection, *: etremely positive ARG in the tumor, **: the most positive ARG in the tumor, \*\*\*: specific positive ARG in the tumor but negative in the liver, and Tf: transferrin.

Example 5

### ANTI-TRANSFERRIN ANTIBODIES

A suspension (1mg/1ml) of the transferrin obtained in Example 1 in physiological saline was prepared. WSA adjuvant was added to the suspension and the obtained mixture was repeatedly injected into the tarsus of a rabbit. After two months, blood was collected and centrifuged after keeping stand to give serum, which was fractionated by electrophoresis or gel filtration. A fraction specifically reacting with transferrin was pooled and lyophilized to give the antibodies.

The antibodies were labeled according to the process conventionally used or described in Example 2.

A reagent for diagnosis was prepared from the labeled antibodies by adding thereto about 100 times excess amount of albumin and maltose to the final concentration of 5% and then lyophilizing the mixture.

Example 6

### QUANTITATIVE DETERMINATION OF THE SERUM TRANSFERRIN

Blood (50µℓ) was collected from mice carrying B-16 melanoma. Serum (20µℓ) was obtained from the collected blood. To the serum was added $^{125}$I-tumor transferrin to the final concentration of 100,000 cpm (specific radioactivity: $1.1 \times 10^4$ dpm/10mg as the labeled transferrin). A portion of the serum was placed on a glass filter having a layer of the antitransferrin antibodies obtained in Example 5 and absorbed on Sephadex G-100 and incubation was performed for 1 hour. The filter was transferred into a centrifuge tube (Centricon) and centrifuged at 5,000 rpm for 1 hour to give a residue on the filter. The residue was washed by re-centrifuging with physiological saline. After a sufficient wash, the filter was transferred into a small test tube and the residue on the filter was analyzed for radioactivity by a scintillation counter.

The result is shown in Table 3.

Table 3

| Serum | healthy mice | tumor mice |
|---|---|---|
| Radioactivity | 98,000±2,500cpm | 35.0±3.8cpm |

0213595

- 18 -

WHAT IS CLAIMED IS;

1. A transferrin which is a soluble protein isolated from a biological source, characterized in that: the said transferrin

    (a) increases in blood level with development of malignant tumor.

    (b) has a molecular weight of about 90,000-100,000, and

    (c) has an isoelectric point of about 5.5-6.7

2. A transferrin as claimed in claim 1, which is produced by an animal having malignant tumor cells embeded therein.

3. A transferrin as claimed in claim 2, in which the said animal is a mammal.

4. A transferrin as claimed in claim 1, in which the said malignant tumor is melanoma.

5. A transferrin as claimed in claim 1 which increases to a rate represented by transferrin/albumin of 0.28 - 30 within 20 days after embedding of malignant tumor cells.

6. A transferrin as claimed in claim 1 which increases to a rate represented by transferrin/albumin of 0.5 - 20 within 20 days after embedding of malignant tumor cells.

7. A transferrin as claimed in claim 1 which increases to a rate represented by transferrin/albumin of 1.0 - 10 within 20 days after embedding of malignant tumor cells.

8. A transferrin as claimed in claim 1, further characterized in that it contains, as amino acid residue,

aspartic acid, threonine, serine, glutamic acid, glycine, alanine, valine, methionine, isoleucine, leucine, tyrosine, phenylalanine, lysine, histidine, arginine and proline.

9. A transferrin as claimed in claim 1, further characterized in that it has an amino acid composition by ninhydrin reaction approximately as follows:

| | |
|---|---|
| Aspartic acid | 67 |
| Threonine | 34 |
| Serine | 35 |
| Glutamic acid | 62 |
| Glycine | 52 |
| Alanine | 51 |
| Valine | 46 |
| Methionine | 4 |
| Isolencine | 20 |
| Leucine | 54 |
| Tyrosine | 21 |
| Phenylalanine | 35 |
| Lysine | 54 |
| Histidine | 19 |
| Arginine | 26 |
| Proline | 47 |

10. A transferrin as claimed in claim 1, having a label selected from a group consisting of isotopes, fluorophores and enzymes.

11. A process for preparation of a transferrin which is a soluble protein isolated from a biological source and

    (a)  increases in blood level with development of malignant tumor,

    (b)  has a molecular weight of about 90,000-100,000, and

    (c)  has a isoelectric point of about 5.5-6.7

said process comprises embedding malignant tumor cells into an animal, feeding the animal, recovering body fluid containing the said transferrin from the animal, separating a fraction containing a protein having molecular weight of about 90,000-100,000 and an isoelectric point of about 5.5-5.7 from the said fluid and recovering the protein from the said fraction.

12. A diagnostic agent for malignant tumor comprising a transferrin as claimed in claim 1 having a label selected from a group consisting of isotopes, fluorophores and enzymes.

13. Antibodies produced in animal in response to an antigen consisting of a transferrin as claimed in claim 1.

14. A kit for detecting malignant tumor comprising a packaged combination of

    a)  a transferrin as claimed in claim 1, and

    b)  antibodies produced in animal in response to an antigen consisting of a transferrin as claimed in claim 1.

15. A kit as claimed in claim 10, comprising further

    c)    solvents for reaction and washing,

    d)    tools and/or apparatuses for reaction and washing, and/or

    e)    means for detecting label.

16. A method of detecting malignant tumors which comprises adding laveled transferrin and antibldy ot a serum sample and measuring the activity of label on the reaction product of the transferrin and antibody.

Fig. 1

| MW (K) | No. | Serum Healthy content | Serum Tumor content | Liver content | Liver ARG | Tumor content | Tumor ARG | Embedded tumor 7th day content | Embedded tumor 14th day content | Injured Liver 7th day content | Injured Liver 14th day content | Note |
|---|---|---|---|---|---|---|---|---|---|---|---|---|
| | 105 | | | | | | | | | | | |
| | 98 | | | | | | | | | | | |
| | 94 | | | | 4.29 | | | | | | | |
| (α₂M) 88 | | | | 9.50 | | | 3.55 | 5.09 | | 4.27 / 12.67 | 9.12 | |
| 85 | | 4.39 | | | 6.36 | | | | 4.83 | | | |
| 77 | | | | 6.23 | | | | 6.44 | 4.83 | 8.84 | 5.94 | |
| 75 | | 15.34 | 38.53 | 5.69 | | 5.54 | 4.46 | 7.55 | 7.16 | 8.06 | 7.69 | * |
| (Tf) 64 | | | | | | | | | | | | |
| (Alb) 60 | | 53.86 | 28.63 | 4.10 | 8.94 | 10.67 | 6.58 | 31.03 | 8.49 | 26.30 | 4.61 | |
| 54 | | 9.36 | 15.50 | 4.68 | 4.24 | 6.52 | 6.59 | | 6.60 | 5.20 | 4.06 | |
| 51 | | | | 4.04 | 4.68 | | | | | 6.15 | 3.00 / 0.05 | |
| 49 | | 5.39 | | 4.07 | | 5.43 | | 6.49 | 8.49 | 3.87 | 4.55 | |
| (Tf) 45 | | | | 5.39 | 10.17 | 6.92 | 16.51 | 8.20 | 10.27 | | 4.46 | ** |
| 43 | | | | 14.46 | | | | | | | 14.71 | |
| 41 | | | | | | | | | | | | |
| 40 | | | | | 5.27 | | | | 4.56 | | | *** |
| 37 | | | | | | 6.50 | 10.08 | | | | | * |
| 35 | | | | | 4.24 | | | | 6.82 | | | |
| 34 | | | | 4.19 | 3.53 | | | | 4.37 | | | |
| 32 | | | | | | | | | | | | |
| 30 | | | | 4.65 | | 5.06 | | | | | | |
| 28 | | | | 4.24 | 4.55 | 3.29 | | | 3.99 | | | |
| 23 | | | | | | 4.60 | | | 3.33 | | | |
| (Gc) 21 | | 4.31 | | | | 3.63 | | | | | | |
| 20 | | | | | | | | | | | | |
| 19 | | | | 3.31 | | | | | | | 3.57 | |
| 18 | | | | | | 3.34 | | 4.32 | | | | |
| 14 | | | | | | | | | 3.64 | | | |
| 10 | | | | 6.22 | 14.87 | 17.83 | 17.39 | 13.91 | 8.82 | 14.80 | 3.19 | |

0213595